# EUROPEAN PATENT SPECIFICATION

(11) **EP 4 648 711 B1**
(45) Date of publication and mention of the grant of the patent: **28.01.2026**
(21) Application number: 24708139.1
(22) Date of filing: 07.02.2024
(51) Int. Cl.: A61C 13/00, G16H 30/00

(54) **SYSTEMS AND METHODS FOR PROVIDING PERSONALIZED VIRTUAL DIGITAL DENTITION OF A PATIENT**
SYSTEME UND VERFAHREN ZUR BEREITSTELLUNG EINES PERSONALISIERTEN VIRTUELLEN DIGITALEN ZAHNERSATZES FÜR EINEN PATIENTEN
SYSTÈMES ET MÉTHODES POUR FOURNIR UNE DENTITION NUMÉRIQUE VIRTUELLE PERSONNALISÉE D'UN PATIENT

(30) Priority: 28.02.2023 EP 23159268
(43) Date of publication of application: 19.11.2025
(73) Proprietor: Digicuto, 13008 Marseille (FR)
(72) Inventor: KOUBI, Stefan, 13008 Marseille (FR); GUREL, Galip, 13008 Marseille (FR); TOURBAH, Karim, 13008 Marseille (FR)
(74) Representative: A.P.I. Conseil
(86) International application number: PCT/EP2024/053111
(87) International publication number: WO 2024/179794

(56) References cited:
- WO-A1-2019/218031
- US-A1- 2021 282 898
- BOYAN YANKOV ET AL: "Software Application for Smile Design Automation Using the Visagism Theory", COMPUTER SYSTEMS AND TECHNOLOGIES 2016, ACM, 2 PENN PLAZA, SUITE 701 NEW YORK NY 10121-0701 USA, 23 June 2016 (2016-06-23), pages 237 - 244, XP058306456, ISBN: 978-1-4503-4182-0, DOI: 10.1145/2983468.2983521
- TENSORFLOW: "Basic classification: Classify images of clothing", TENSORFLOW TUTORIALS, 7 July 2022 (2022-07-07), pages 1 - 18, XP093064794, Retrieved from the Internet <URL:https://www.tensorflow.org/tutorials/keras/classification> [retrieved on 20230718]

## Description

### Technical Field

The subject application relates to systems and methods for providing personalized virtual digital dentition of a patient. Similar systems are known from Yankov, B., Iliev, G.V., Filchev, D., Gurel, G., Paolucci, B., Shayder, A., & Misheva, I. (2016). Software Application for Smile Design Automation Using the Visagism Theory. Proceedings of the 17th International Conference on Computer Systems and Technologies 2016.

### Background Art

It is known that facial attractiveness influences personality development and social interaction.

Among others, the smile of an individual is considered as an important element of facial attractiveness.

Principles of a pleasing smile have been described in the dental literature by numerous authors who suggest various parameters for optimal facial and dental composition.

Recently, it has been proposed to establish, for individuals, a relationship between human personality trait and dentofacial aesthetics.

Indeed, it is believed that as personality is unique to an individual, it could be used to design a customized smile.

However, today, human personality traits are determined by questionnaires that are time consuming to administer and process.

It is an object of the present subject application to provide a novel technique to overcome drawbacks typically associated with the use of questionnaires in the design of a customized smile.

### Summary of Subject application

The subject application provides systems and methods for providing personalized virtual digital dentition of a patient, as described in the accompanying claims.

Dependent claims describe specific embodiments of the subject application.

These and other aspects of the subject application will be apparent from an elucidated based on the embodiments described hereinafter.

### Brief Description of Drawings

Further details, aspects and embodiments of the subject application will be described, by way of example only, with reference to the drawings. In the drawings, like reference numbers are used to identify like or functionally similar elements. Elements in the figures are illustrated for simplicity and clarity and have not necessarily been drawn to scale.
Figure 1 shows a block diagram of a system according to the subject application.
Figure 2 shows a segmented incisor, according to a first embodiment.
Figure 3 shows a segmented incisor, according to a second embodiment.
Figure 4 shows a schematic flow diagram of method according to the subject application.

### Description of Embodiments

Because the illustrated embodiments of the subject application may, for the most part, be composed of components known to the skilled person, details will not be explained in any greater extent than that considered necessary for the understanding and appreciation of the underlying concepts of the subject application, in order not to obfuscate or distract the reader from the teachings of the subject application.

Inventors have found a way of designing a personalized virtual digital dentition of a patient that is based only on a digital image of the front face of the patient.

The subject application relates to a system implemented by a computer for providing a personalized virtual digital dentition of a patient.

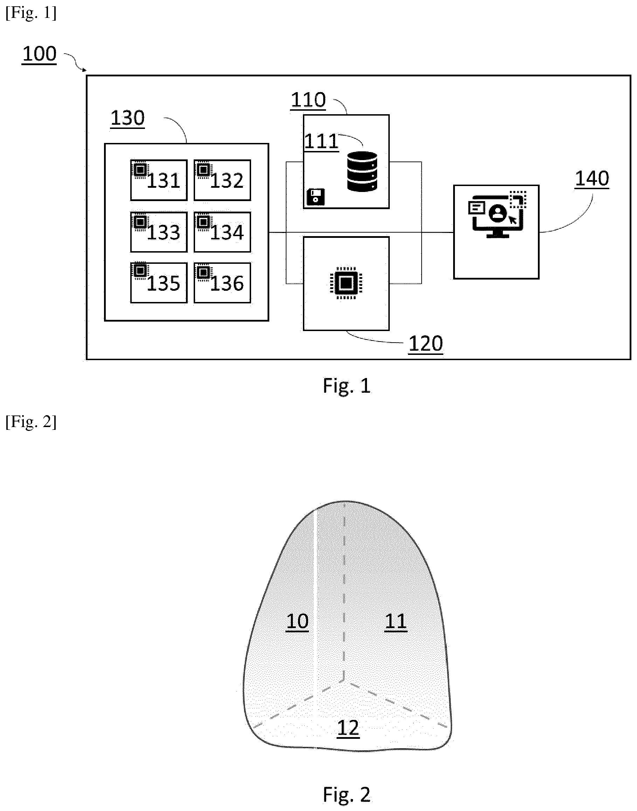

Referring to figure 1, the system 100 comprises at least one memory 110, processor-based acquiring means 120 and processor-based computer vision means 130, which are operatively coupled to each other.

In other words, one should understand that the system 100 may comprise more than one memory 110.

The memory 110 is of known type (i.e., any suitable computer-accessible or non-transitory storage medium for storing computer program instructions, such RAM, SDRAM, DDR SDRAM, RDRAM, SRAM, ROM, magnetic media, optical media and the like) and therefore will not be further detailed.

In the subject application, the memory 110 is configured for storing computer program instructions for execution by a processor.

The program instructions refer to the executable code that is stored in the memory and will be executed by the processor. The program instructions are typically written in a programming language such as C, C++, Java, Python, etc. When executed, the program instructions cause the processor to perform specific computational tasks and operations.

The processor is the hardware component that executes the program instructions stored in the memory. It is typically a central processing unit (CPU) composed of complex logic and electronic circuitry. The processor fetches instructions from memory, decodes them, and executes them by performing arithmetic, logical, control, and other types of operations. There are various types of processors based on different architectures such as x86, ARM, RISC-V, etc. Modern processors employ techniques like pipelining, superscalar execution, branch prediction, etc. to improve performance. In summary, the processor is the engine that runs the program instructions stored in the memory.

The memory 110 is further configured for storing a plurality of predetermined reference cranio-facial vectors.

As used herein, the term "vector" is defined as being any suitable data structure that holds a number of consecutive data elements.

In a first example, a standard array data structure in programming languages like C or Java represents a typical implementation of a vector. An array declares consecutive memory locations to store elements of the same data type, like integers, characters etc. The elements are stored adjacently in memory and can be accessed via indexed addressing. The size or length of the array indicates the maximum number of data elements it can sequentially hold.

In a second example, a linked list data structure also exhibits the properties of a vector. A linked list contains nodes where each node stores a data value and a pointer or reference to the next node. This chain of references logically forms a sequence of consecutive data elements, even though the nodes may not be physically adjacent in memory. The linked list size denotes the number of data nodes it links together.

In a third example, a multidimensional array extends the basic array to organize data along multiple dimensions. A 2D array declares a matrix storing rows and columns of elements in a contiguous grid layout. Though stored in a multidimensional format, the elements are arranged sequentially row by row.

In a fourth example, a data buffer is commonly used to temporarily store a continuous sequence of bytes or network packets. The buffer is an in-memory data structure that provides sequential storage and access of consecutive data.

In a fifth example, a data stream represents a continuous flow of data elements made available over time. A stream sequentially passes ordered data from a source to a destination in a first-in-first-out manner.

In particular, each predetermined reference cranio-facial vector is associated with at least one type of predetermined human personality trait.

As used herein, the term "human personality trait" is defined as personal characteristics of an individual that are revealed in a particular pattern of behavior in a variety of situations.

For instance, in the dentistry field, it is commonly admitted that an individual's personality is formed by a unique combination of four types of traits:
- « choleric/strong » corresponding to individuals characterized by strong leadership qualities and fearlessness. They have a rectangular face with well-defined angles. The maxillary anterior teeth are positioned with their long axes perpendicular to the horizontal plane. These individuals have dominant rectangular central incisors. The connection line of the embrasures is horizontal between the central and lateral incisors,
- « sanguine/dynamic » corresponding to individuals that are very active, communicative, and extroverted. They have an angular face. The long axes of the maxillary anterior teeth are inclined slightly distally. The central incisors are usually triangular or trapezoidal. The connection lines of the embrasures and the incisal plane are ascendants from the medial line,
- « melancholic/delicate » corresponding to individuals characterized by gentleness and abstract thinking. This type of individual has an oval face with rounded features. The long axes of the maxillary anterior teeth are distally inclined. The central incisors are usually oval. The connection lines of the embrasures descend from the medial line, creating an inverted incisal plane, and
- « phlegmatic/calm » corresponding to individuals that are gentle, discreet and diplomatic. They have a round or a square face. The maxillary anterior teeth have their long axes perpendicular to the horizontal plane. The central incisors are square and small. The connection line of the embrasures is straight.

However, other taxonomy of individual's personality traits may be contemplated, without requiring any substantial modification of the subject application.

In particular, one could consider combining reference cranio-facial vectors associated with different human personality traits to create combined reference cranio-facial vectors.

For instance, a weighted average of the choleric and sanguine vectors can yield a reference vector for a personality that is both dominant and extraverted. The weights modulate the relative influence of each trait. Similarly, combining the melancholic and phlegmatic vectors can give a reference for a calm, introverted personality.

In essence, blending reference vectors associated with different traits allows creating cranio-facial vectors that model nuanced, real-life personalities rather than simplistic archetypes. The composite vectors provide more flexibility in profiling the subtleties and complexities of human personality based on facial features.

This technique of synthesizing reference vectors is beneficial in the dentistry field where modeling the diversity and richness of human personality traits is important. The ability to finely tune personality types by tuning vector weights allows generating a wide spectrum of facial profiles via a combinatorial approach.

In practice, the personality trait of an individual can be determined through the use of a questionnaire (e.g., self-administered, researcher-administered or doctor-administered) that asks individuals to mark some statements that describe their own behavior.

However, other methods for determining the personal trait of an individual may be contemplated, without requiring any substantial modification of the subject application.

In a first example, an expert could analyze verbal and non-verbal language, social interactions and behavior of the individual to evaluate personality characteristics. Projective tests like the Rorschach inkblot test reveal facets of personality through interpretation of ambiguous visual stimuli.

In a second example, personality traits could be predicted by examining social media presence and digital footprints which provide clues to certain traits.

In a third example, biometric sensors can detect physiological signals like heart rate variability that correlate with specific personalities.

In a fourth example, machine learning algorithms applied on behavioral data sets could automatically predict personality traits based on patterns and correlations. The predictions can be calibrated to match conventional questionnaire-based assessments.

Also, each predetermined reference cranio-facial vector comprises a set of reference cranio-facial features.

As used herein, the term "cranio-facial features" is defined as being any measurable characteristic that can be derived from a substantially front face view of an individual.

In a first example, cranio-facial features comprise location and geometry (e.g., contours, angles, sizes) of anatomical parts of the face of an individual, such as the nose, the mouth, the eyes, the pupils, the eyebrows, the chin, the cheeks, and the forehead.

In a second example, skin texture, facial hair and pigmentation could provide complementary information for analysis.

In a third example, aspects like skull shape and Adam's apple that are not directly visible on the face also contribute to cranio-facial features.

In a fourth example, combinations of multiple facial elements such as inter-eye distance and nose width could yield new composite characteristics.

In a fifth example, tracking temporal evolution of certain facial features could reveal relevant patterns for analysis.

However, other anatomical parts or combination of anatomical parts of a front face of an individual may be contemplated, without requiring any substantial modification of the subject application.

In the subject application, the memory 110 is further configured for storing a teeth database 111.

As used herein, the term "database" is defined as being any suitable data storage system such as a relational database (e.g., an object-relational database), a triple store, a hierarchical data store, or any suitable combination thereof.

The teeth database 111 comprises 2D digital images of individual teeth.

As used herein, the term "2D digital image" is defined as a two-dimensional visual representation of an individual tooth that is captured and stored in digital form. It consists of an array of picture elements (pixels) organized on a 2D grid, where each pixel is defined by color values like red, green and blue. The image resolution (pixels per unit length) determines the level of detail and fidelity. The image file format (PNG, JPEG, etc.) defines how the pixel color information is encoded and compressed for storage. 2D digital images enable computerized analysis and processing of shapes, textures and colors, and are widely used in fields like computer vision and image processing. They provide a quantitative representation amenable to automated interpretation and manipulation by algorithms.

In particular, each 2D digital image is associated with one type of tooth (i.e., incisor, canine, premolar, molar) and at least one predetermined human personality trait.

In other words, one should understand that each 2D digital image may be associated with one type of tooth and more than one predetermined human personality trait.

Therefore, in the database 111, each data record corresponds to a single 2D digital image of an individual tooth which is associated with one type of tooth and at least one predetermined human personality trait.

In a first embodiment of the database 111, each data record corresponds to a single 2D digital image of an individual tooth which is associated with one type of tooth and one predetermined human personality trait.

In a second embodiment of the database 111, each data record corresponds to a single 2D digital image of an individual tooth which is associated with one type of tooth and more than one predetermined human personality trait.

This second embodiment aims at producing different tooth anatomies that extend beyond the standard tooth shapes.

In particular, in that second embodiment, each data record corresponds to a 2D digital image which is a combination of segmented portions of 2D digital images respectively associated with one predetermined human personality trait.

Indeed, each 2D digital image associated with one type of tooth and one predetermined human personality trait (such as those of the first embodiment of the database 111) may be segmented into two or more portions. Then, the segmented portions associated with one type of tooth and a plurality of predetermined human personality traits can be combined to form a combined 2D digital image. Therefore, the combined 2D digital image will be associated with the plurality of predetermined human personality traits.

Let's take an example with an incisor type of tooth.

In a first variation of the example, as illustrated in figure 2, each 2D digital image associated with an incisor can be segmented into three portions such as a mesial portion 10, a distal portion 11 and an incisal portion 12.

In that first variation of the example, for instance, a combined 2D digital image can correspond to a combination of a mesial portion 10 associated with a « choleric/strong » human personality trait, a distal portion 11 associated with a « sanguine/dynamic » human personality trait and an incisal portion 12 associated with a « melancholic/delicate » human personality trait.

However, other combinations may be contemplated, without requiring any substantial modification of the subject application.

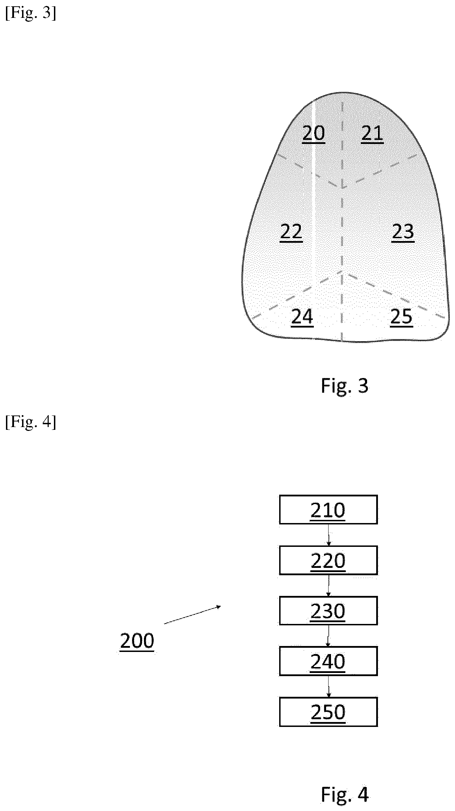

In a second variation of the example, as illustrated in figure 3, each 2D digital image associated with an incisor can be segmented into six portions such as a mesial cervical portion 20, a distal cervical portion 21, a mesial body portion 22, a distal body portion 23, a mesial incisal portion 24 and a distal incisal portion 25.

In that second variation of the example, for instance, a combined 2D digital image can correspond to a combination of a mesial cervical segmented portion 20 associated with a « phlegmatic/calm » human personality trait, a distal cervical segmented portion 21 associated with a « phlegmatic/calm » human personality trait, a mesial body segmented portion 22 associated with a « choleric/strong » human personality trait, a distal body segmented portion 23 associated with a « choleric/strong » human personality trait, a mesial incisal segmented portion 24 associated with a « sanguine/dynamic » human personality trait and a distal incisal segmented portion 25 associated with a « sanguine/dynamic » human personality trait.

However, other combinations may be contemplated, without requiring any substantial modification of the subject application.

In a variation of the first and second embodiments of the database 111, each data record may also be associated with at least one particular visual characteristic of the single 2D digital image, such as the color or the tint of the tooth.

However, other measurable characteristics of 2D digital images may be contemplated, without requiring any substantial modification of the subject application.

In a first example, image texture describing surface variations and patterns can be quantified.

In a second example, color properties like brightness, contrast and saturation can be measured.

In a third example, geometric shape outlines and contours delineating objects can be analyzed.

In a fourth example, statistical features like color histograms and co-occurrence matrices can be extracted.

In a fifth example, blur, noise and artifacts can be quantified to assess image quality.

In the subject application, the memory 110 is further configured for storing a first predetermined sequence.

As used herein, the term "predetermined sequence" is defined as a specific order of elements established in advance according to defined rules or criteria. It represents a particular ordering defined prior to use. The predetermined sequence may take various forms - an ordered list, a ranking, a chronology, etc. It is typically used to define the arrangement in which certain events should occur or actions should be executed. The predetermined sequence introduces logic, consistency and reproducibility in a process by predefining the sequence of operations. In other words, a predetermined sequence prescribes a definite succession or flow based on pre-set ordering rules rather than real-time determinations.

In particular, the first predetermined sequence is representative of a type of tooth order, in which 2D digital images of individual teeth are to be considered for positioning on a 2D image.

As used herein, the term "type of tooth order" is defined as corresponding to an order in which things occur based on the type of tooth.

In a first embodiment of the first predetermined sequence, the first predetermined sequence is ordered according to the following series: incisors, canines, premolars and molars.

In a second embodiment of the first predetermined sequence, the first predetermined sequence is ordered according to the following series: canines, incisors, premolars and molars.

However, other sequencing of the teeth according to their type may be contemplated, without requiring any substantial modification of the subject application.

The processor-based acquiring means 120 is of known type (i.e., any suitable processor capable of executing memory-stored instructions, such as a microprocessor, uniprocessor, a multiprocessor, and the like) and therefore will not be further detailed.

Still in the subject application, the processor-based acquiring means 120 is configured for acquiring at least one digital image of the front face of the patient smiling.

In other words, one should understand that the processor-based acquiring means 120 may acquire more than one digital image.

In a first implementation of the processor-based acquiring means 120, the acquired digital image is a 2D digital image.

In a second implementation of the processor-based acquiring means 120, the acquired digital image is a 2D digital representation of a cross-section through a 3D digital image.

As used herein, the term "3D digital image" refers to a three-dimensional visual representation of an individual tooth that is digitally captured and stored. It encodes depth information in addition to width and height dimensions. Each pixel has 3D coordinates (x, y, z). 3D imaging techniques include stereovision, structured light, photogrammetry, 3D scanners etc. 3D images allow reconstructing object surfaces and shapes for observation from different viewpoints. They find applications in 3D modeling, augmented reality, robotics etc. 3D file formats like OBJ, PLY, STL are used to store and exchange the 3D data. They provide a quantitative model of an object's 3D geometry and appearance that can be computationally processed and analyzed.

Further, the acquired digital image is such that at least a portion of the dentition of the patient can be seen.

In other words, one should understand that the acquired digital image may exhibit a substantial part of the dentition of the patient.

Yet, in the subject application, the processor-based computer vision means 130 comprises processor-based teeth detecting means 131, processor-based cranio-facial detecting means 132, processor-based ranking means 134, processor-based positioning means 135 and processor-based comparing means 133, which are operatively coupled to each other.

The processor-based computer vision means 130 is of known type (i.e., any suitable processor capable of executing memory-stored instructions implementing computer vision techniques, such as a microprocessor, uniprocessor, a multiprocessor, and the like) and therefore will not be further detailed.

The processor-based teeth detecting means 131 are configured for detecting at least one tooth in the digital image of the front face of the patient.

In other words, one should understand that the processor-based teeth detecting means 131 may detect more than one tooth in the digital image of the front face of the patient.

In an embodiment of the processor-based teeth detecting means 131, the processor-based teeth detecting means 131 are further configured for detecting a mouth opening boundary region formed between the upper lip, the lower lip, the oral commissures of the mouth of the patient.

Further, in the embodiment of the processor-based teeth detecting means 131, the processor-based teeth detecting means 131 detect the tooth within the mouth opening boundary region.

In an implementation of the embodiment of the processor-based teeth detecting means 131, the system 100 further comprises a user interface 140.

As used herein, the term "user interface" is defined as being any suitable software rendering system for providing to and/or receiving information from a user of the user interface via a display device. The user interface may be based on one more of the following interactions: visual, graphical, tactile, audible, sensory or the like.

In the subject application, the user interface 140 is configured for allowing, through user interaction, digital modification of at least one point defining the mouth opening boundary region.

In other words, one should understand that the user interface 140 may allow digital modification of more than one point defining the mouth opening boundary region.

In the subject application, the processor-based cranio-facial detecting means 132 are configured for detecting a set of cranio-facial features of the patient based on the digital image of the front face of the patient.

The processor-based cranio-facial detecting means 132 are further configured for generating a patient cranio-facial vector based on the detected set of cranio-facial features.

In the subject application, the processor-based comparing means 133 are configured for comparing the patient cranio-facial vector with the plurality of predetermined reference cranio-facial vectors.

The processor-based comparing means 133 are further configured for calculating a plurality of similarity measures that are greater than a predetermined similarity threshold.

In particular, a similarity measure is representative of a matching similarity between the patient cranio-facial vector and one predetermined reference cranio-facial vector.

In other words, the number of similarity measures depends on the number of predetermined reference cranio-facial vectors.

In a first example, where there are two predetermined reference cranio-facial vectors, the processor-based comparing means 133 calculates two similarity measures.

In a second example, where there are four predetermined reference cranio-facial vectors, the processor-based comparing means 133 calculates four similarity measures.

In a first particular implementation of the processor-based comparing means 133, the similarity measures correspond to matching percentages.

In a second particular implementation of the processor-based comparing means 133, the processor-based comparing means 133 keeps only the similarity measures that are greater than a predetermined similarity threshold and discards the others.

In an example of the second particular implementation of the processor-based comparing means 133, in relation to the first particular implementation of the processor-based comparing means 133, the predetermined similarity threshold is greater than 0%, preferably greater than 2%, more preferably greater than 5%, most preferably greater than 10%.

In the subject application, the processor-based ranking means 134 are configured for ranking the similarity measures according to a given ranking order.

In a first embodiment of the processor-based ranking means 134, the given ranking order is an order from smaller to larger order.

In a second embodiment of the processor-based ranking means 134, the given ranking order is an order from larger to smaller order.

However, other ranking orders may be contemplated, without requiring any substantial modification of the subject application.

In a first example, ranking could be based on the category or type of the teeth associated with the similarity measures.

In a second example, ranking could be based random or pseudo-random ranking.

In the subject application, the processor-based positioning means 135 are configured for overlaying, on the digital image of the front face of the patient, the detected tooth with the corresponding 2D digital image of individual tooth, thereby generating a first personalized virtual 2D digital dentition of the patient.

In other words, one should understand that the processor-based positioning means 135 may overlay of more than one 2D digital image of individual tooth.

As used herein, the term "overlaying" is defined as combining two or more images by placing one image on top of another. The topmost image, called a layer, is overlaid onto the background image. The pixels of the top layer partially or fully cover the pixels of the background layer. This allows merging elements from different sources into a single composite image. Overlaying can be done using various blending modes that determine pixel interactions between the layers. It is commonly used in computer graphics and image processing for assembling diverse visual elements. In summary, overlaying refers to stacking image elements hierarchically through layered transparent or semi-transparent arrangements.

In particular, the overlaying is based on the first predetermined sequence and the ranked similarity measures.

In practice, the processor-based positioning means 135 determine, according to the first predetermined sequence, in which order the overlaying will be performed.

Further, the processor-based positioning means 135 selects, from the teeth database 111, at least one 2D digital image of individual tooth based on the ranked similarity measures.

Finally, the processor-based positioning means 135 overlay the detected tooth with selected 2D digital image of individual tooth.

In order to illustrate the functioning of the processor-based positioning means 135, let's take an example in which,
- the first predetermined sequence is ordered according to the following series: incisors, canines, premolars and molars,
- the given ranking order is an order from larger to smaller order, and
- the processor-based comparing means 133 calculated the four similarity measures, expressed in percentages, for a given unknown set of cranio-facial features of a human face:
   - « choleric/strong »: 25%
   - « sanguine/dynamic »: 37.5%
   - « melancholic/delicate »: 34%
   - « phlegmatic/calm »: 3.5%

In a first implementation of the example, the processor-based positioning means 135 can overlay one type of tooth with respect to one similarity measure.

In that case, the processor-based positioning means 135 may first overlay the incisors (since "incisors" are positioned in the first position of the first predetermined sequence) with a 2D digital image of individual tooth associated with the « sanguine/dynamic » human personality trait (since « sanguine/dynamic » human personality trait is the largest percentage of similarity measures).

Then, in the first implementation of the example, the processor-based positioning means 135 may overlay the canines (since "canines" are positioned in the second position of the first predetermined sequence) with a 2D digital image of individual tooth associated with the « melancholic/delicate » human personality trait (since « melancholic/delicate » human personality trait is the second largest percentage of similarity measures).

Further, in the first implementation of the example, the processor-based positioning means 135 may overlay the premolars (since "premolars" are positioned in the third position of the first predetermined sequence) with a 2D digital image of individual tooth associated with the « choleric/strong » human personality trait (since « choleric/strong » human personality trait is the third largest percentage of similarity measures).

Finally, in the first implementation of the example, the processor-based positioning means 135 may overlay the molars (since "molars" are positioned at the last position of the first predetermined sequence) with a 2D digital image of individual tooth associated with the « phlegmatic/peaceful » human personality trait (since « phlegmatic/peaceful » human personality trait is the latest percentage of similarity measures).

In a second implementation of the example, the processor-based positioning means 135 can overlay one or more type of tooth with respect to one similarity measure.

In particular, the number of types of tooth associated with one similarity measure can be predetermined.

In that case, the processor-based positioning means 135 may first overlay the incisors and the canines (since "incisors" and "canines" are positioned in the first and second positions of the first predetermined sequence) with a 2D digital images of individual tooth associated with the « sanguine/dynamic » human personality trait, respectively (since « sanguine/dynamic » human personality traits is the largest percentage of similarity measures).

Then, in the second implementation of the example, the processor-based positioning means 135 may overlay the premolars (since "premolars" are positioned in the third position of the first predetermined sequence) with a 2D digital image of individual tooth associated with the « melancholic/delicate » human personality trait (since « melancholic/delicate » human personality trait is the second largest percentage of similarity measures).

Finally, in the second implementation of the example, the processor-based positioning means 135 may overlay the molars (since "molars" are positioned at the last position of the first predetermined sequence) with a 2D digital image of individual tooth associated with the « choleric/strong » human personality trait (since « choleric/strong »human personality trait is the third largest percentage of similarity measures).

In that case, the processor-based positioning means 135 does not use the « phlegmatic/calm » human personality trait for overlaying a type of tooth.

In a third implementation of the example, the processor-based positioning means 135 can overlay one type of tooth with respect to more than one similarity measure.

In that case, the processor-based positioning means 135 may first overlay the incisors (since "incisors" are positioned in the first position of the first predetermined sequence) with a 2D digital image of individual tooth associated with the « sanguine/dynamic » human personality trait and with the « melancholic/delicate » human personality trait (since « sanguine/dynamic » human personality trait and « melancholic/delicate » human personality trait are the two largest percentages of similarity measures).

Then, in the third implementation of the example, the processor-based positioning means 135 may overlay the canines (since "canines" are positioned in the second position of the first predetermined sequence) with a 2D digital image of individual tooth associated with the « choleric/strong » human personality trait and the « phlegmatic/calm » human personality trait (since
« melancholic/delicate » human personality trait and the
« phlegmatic/calm »human personality trait are the smallest percentages of similarity measures).

In that case, the processor-based positioning means 135 does not use the human personality traits for overlaying the premolars and the molars. This could correspond to a situation where only the incisors and the canines need to be overlaid.

In the third implementation of the example, it was considered that, for a given type of tooth, each 2D digital image is associated with two predetermined human personality traits.

However, as explained above, for a given type of tooth, each 2D digital image may be associated with two or more predetermined human personality traits, without requiring any substantial modification of the subject application.

In a first embodiment of the subject application, the processor-based computer vision means 130 further comprise processor-based dental arch trajectory detecting means 136.

The processor-based dental arch trajectory detecting means 136 is configured for detecting a trajectory of the dental arch of the patient in the digital image of the front face of the patient.

As used herein, the term "trajectory of the dental arch of the patient in the digital image of the front face" refers to the shape described by the alignment of teeth along the jawline in the facial image. It follows the natural curvature of the upper and/or lower jaw containing the teeth. It can be represented by a continuous contour tracing the positioning of teeth along the dental arch. Detecting it delineates the region occupied by teeth in the facial image. It provides important cues about the teeth and jaw layout of the patient. Its analysis yields key insights for digitally reconstructing the teeth into the facial image. In summary, it captures the dental arch form defined by the teeth arrangement along the jaw in the front view of the face.

Further, in the first embodiment of the subject application, the processor-based positioning means 135 are further configured for modifying at least one parameter (e.g., angle, width, length, height, position, curvature, scale, symmetry) of the dental arch trajectory of the patient, based on at least one similarity measure.

In other words, one should understand that the processor-based positioning means 135 may modify more than one parameter of the dental arch trajectory of the patient.

In a second implementation of the first embodiment of the subject application, the dental arch trajectory of the patient is modeled as a Beta function characterized by the depth and the width of the dental arch at different locations in the mouth (e.g., at the second molar region, at the canine molar region and any combination thereof).

In that case, modifying one parameter of the dental arch trajectory could correspond to modifying the depth and/or the width of the Beta function.

In a third implementation of the first embodiment of the subject application, with respect to the second implementation of the example, described above, which illustrates the functioning of the processor-based positioning means 135, the similarity measure used for modifying at least one parameter of the dental arch trajectory of the patient is one that is not used by the processor-based positioning means 135 for overlaying a type of tooth.

Further, in the first embodiment of the subject application, the processor-based positioning means 135 overlay the detected tooth with the corresponding 2D digital image of individual tooth, along the modified dental arch trajectory of the patient.

In a second embodiment of the subject application, the processor-based comparing means 133 comprise a machine learning classifier.

As used herein, the term "machine learning classifier" refers to an artificial intelligence algorithm capable of categorizing data into predefined classes or categories. It is trained on labeled data to learn to recognize patterns and make classification decisions. Machine learning classifiers include neural networks, decision trees, support vector machines, etc. They analyze input data features to predict the most likely class. Machine learning classifiers are used for image recognition, anomaly detection, segmentation, medical diagnosis, etc. They enable automating complex classification and decision-making tasks. In summary, a machine learning classifier is a model that uses statistical learning on training data to perform automatic classification of new data based on learned parameters.

In practice, the machine learning classifier is trained using, as training data, a plurality of sets of cranio-facial features that have been extracted from a plurality of images of the front face of human faces.

Further in the second embodiment of the subject application, the machine learning classifier is configured for predicting the likelihood that the set of cranio-facial features of a human face is similar to each of the set of reference cranio-facial features of the plurality of predetermined reference cranio-facial vectors.

Yet in the second embodiment of the subject application, the machine learning classifier is further configured for outputting similarity measures representative of a matching similarity between an inputted set of cranio-facial features of a human face and each of the set of reference cranio-facial features of the plurality of predetermined reference cranio-facial vectors.

In a third embodiment of the subject application, the teeth database 111 further comprises 3D digital images of individual teeth.

In particular, each 3D digital image is associated with one type of tooth and at least one predetermined human personality trait.

In other words, one should understand that each 3D digital image may be associated with one type of tooth and more than one predetermined human personality trait.

Therefore, in the database 111, each data record corresponds to a single 3D digital image of an individual tooth which is associated with one type of tooth and at least one predetermined human personality trait.

In a first embodiment of the database 111, each data record corresponds to a single 3D digital image of an individual tooth which is associated with one type of tooth and one predetermined human personality trait.

In a second embodiment of the database 111, each data record corresponds to a single 3D digital image of an individual tooth which is associated with one type of tooth and more than one predetermined human personality trait.

This second embodiment aims at producing different tooth anatomies that extend beyond the standard tooth shapes.

In particular, in that second embodiment, each data record corresponds to a 3D digital image which is a combination of segmented portions of 3D digital images respectively associated with one predetermined human personality trait.

Indeed, each 3D digital image associated with one type of tooth and one predetermined human personality trait (such as those of the first embodiment of the database 111) may be segmented into two or more portions. Then, the segmented portions associated with one type of tooth and a plurality of predetermined human personality traits can be combined to form a combined 3D digital image. Therefore, the combined 3D digital image will be associated with the plurality of predetermined human personality traits.

Let's take an example with an incisor type of tooth.

In a first variation of the example, each 3D digital image associated with an incisor can be segmented into three portions such as a mesial portion, a distal portion and an incisal portion.

In that first variation of the example, for instance, a combined 3D digital image can correspond to a combination of a mesial segmented portion associated with a « phlegmatic/calm » human personality trait, a distal segmented portion associated with a « sanguine/dynamic » human personality trait and an incisal segmented portion associated with a « melancholic/delicate » human personality trait.

However, other combinations may be contemplated, without requiring any substantial modification of the subject application.

In a second variation of the example, each 3D digital image associated with an incisor can be segmented into six portions such as a mesial cervical portion, a distal cervical portion, a mesial body portion, a distal body portion, a mesial incisal portion and a distal incisal portion.

In that second variation of the example, for instance, a combined 3D digital image can correspond to a combination of a mesial cervical segmented portion associated with a « melancholic/delicate » human personality trait, a distal cervical segmented portion associated with a « phlegmatic/calm » human personality trait, a mesial body segmented portion associated with a « melancholic/delicate » human personality trait, a distal body segmented portion associated with a « phlegmatic/calm » human personality trait, a mesial incisal segmented portion associated with a « melancholic/delicate » human personality trait and a distal incisal segmented portion associated with a « phlegmatic/calm » human personality trait.

However, other combinations may be contemplated, without requiring any substantial modification of the subject application.

In a variation of the first and second embodiments of the database 111, each data record may also be associated with at least one particular visual characteristic of the single 3D digital image, such as the color or the tint of the tooth.

However, other measurable characteristics of 3D digital images may be contemplated, without requiring any substantial modification of the subject application.

In a first example, image texture describing surface variations and patterns can be quantified.

In a second example, color properties like brightness, contrast and saturation can be measured.

In a third example, geometric shape outlines and contours delineating objects can be analyzed.

In a fourth example, statistical features like color histograms and co-occurrence matrices can be extracted.

In a fifth example, blur, noise and artifacts can be quantified to assess image quality.

Further, in the third embodiment of the subject application, the memory 110 is further configured for storing a second predetermined sequence similar to the first predetermined sequence, as already described above.

In particular, the second predetermined sequence is representative of a type of tooth order, in which 3D digital images of individual teeth are to be considered for positioning on a 2D image.

Yet, in the third embodiment of the subject application, the processor-based positioning means 135 are further configured for overlaying, on the digital image of the front face of the patient, the detected tooth with a 2D digital representation of a cross-section through the corresponding 3D digital image of individual tooth, thereby generating a second personalized virtual 2D digital dentition of the patient.

In other words, one should understand that the processor-based positioning means 135 may overlay a 2D digital representation of a cross-section through more than one 3D digital image of individual tooth.

In particular, the overlaying is based on the second predetermined sequence and the ranked similarity measures.

In an implementation of the third embodiment of the subject application, the processor-based acquiring means 120 are further configured for acquiring a 3D intraoral scan of the dentition of the patient.

Further, in the implementation of the third embodiment of the subject application, the processor-based teeth detecting means 131 are further configured for detecting at least one tooth in the 3D intraoral scan.

In other words, one should understand that the processor-based teeth detecting means 131 detect more than one tooth in the 3D intraoral scan.

Still further, in the implementation of the third embodiment of the subject application, within the teeth database 111, each 3D digital image is associated with one corresponding 2D digital image.

Yet, in the implementation of the third embodiment of the subject application, the processor-based positioning means 135 are further configured for overlaying the 3D intraoral scan with the first or second personalized virtual 2D digital dentition.

Besides, in the implementation of the third embodiment of the subject application, the processor-based positioning means 135 are further configured for replacing the tooth detected in the 3D intraoral scan by the 3D digital image associated with the first or second personalized virtual 2D digital dentition, thereby generating a personalized virtual 3D digital dentition.

The subject application also relates to a method of training a classifier for predicting the likelihood that a set of cranio-facial features of a human face is similar to each of the set of reference cranio-facial features of a plurality of predetermined reference cranio-facial vectors.

As used herein, the term "training" refers to the process of enabling a machine learning algorithm to learn to perform classification tasks. During training, the classifier is exposed to labeled input data for which the true classification is known. By analyzing these training examples, the classifier **iteratively** adjusts the weights and parameters of its model to minimize classification error. The larger and more representative the training dataset, the better the performance. Techniques like deep learning allow training complex classifiers on large data volumes. Training is a critical step in developing accurate classifiers. In summary, training involves tuning the classifier using correctly labeled data until it can reliably map new inputs to their correct output classes.

As shown in figure 4, the method 200 is executed by a processor.

In step 210, the processor collects a plurality of sets of cranio-facial training features, each being associated with at least an image of the front face of a human, as already described above.

In a first implementation of step 210, each set of cranio-facial training features comprises a combination of at least two measurable characteristics, similar to those described above, that can be derived from a substantially front face view of an individual.

In a second implementation of step 210, the collected plurality of sets of cranio-facial training features comprises a plurality of positive sets of cranio-facial training features and a plurality of negative sets of cranio-facial training features.

As generally known in the field of machine learning, the positive sets of cranio-facial training features comprise examples labeled as being associated with one or more predetermined personality traits. They represent cases where the facial features match expected personality traits. The negative sets of cranio-facial training features comprise examples labeled as not being associated with the predetermined personality traits. They represent cases where the facial features do not exhibit expected personality trait patterns. These two types of training sets enable the classifier to learn to differentiate between positive and negative cases in relation to expected associations with personality traits.

In particular, in the plurality positive sets of cranio-facial training features, each set of cranio-facial training features has been identified as being associated with at least one type of predetermined human personality trait.

Also, in the plurality of negative sets of cranio-facial training features, each set of cranio-facial training features has been identified as not being associated with at least one type of predetermined human personality trait.

In an example of the second implementation of step 210, the number of positive sets of cranio-facial training features is substantially equal to the number of negative sets of cranio-facial training features.

In step 220, the processor generates a plurality of cranio-facial training vectors based on the plurality of sets of cranio-facial training features, as already described above.

In step 230, the processor obtains a plurality of predetermined reference cranio-facial vectors, as already described above.

In particular, each predetermined reference cranio-facial vector is associated with at least one type of predetermined human personality trait, as already described above.

Also, each predetermined reference cranio-facial vector comprises a set of reference cranio-facial features, as already described above.

In step 240, the processor calculates a plurality of similarity measures, each being representative of a matching similarity between one set of cranio-facial training features and one predetermined reference cranio-facial vector, as already described above.

In a particular implementation of step 240, the similarity measures correspond to matching percentages.

Finally, in step 250, the processor applies the plurality of sets of cranio-facial training features and the plurality of similarity measures to a machine-learning algorithm to generate a trained classifier configured for outputting similarity measures representative of a matching similarity between, on the one hand, an input cranio-facial vector, and on the other hand, each of the plurality of predetermined reference cranio-facial vectors.

In a first implementation of step 250, the classifier is configured for outputting the similarity measures that are greater than a predetermined similarity threshold.

In a second implementation of step 250, the machine learning algorithm comprises a machine learning algorithm selected among random forests, support vector machine and neural networks.

The subject application also relates to a computer-readable medium having stored thereon computer instructions which when executed, by a processor, perform the method 200 as described above.

As used herein, the term "computer-readable medium" refers to any storage device from which data can be read and processed by a computing system. It includes hard drives, USB sticks, CD/DVDs, flash memory, memory cards, etc. The data may represent program instructions, information files, multimedia content, etc. The computer accesses the medium and reads the data using standard protocols and interfaces. Compared to paper-based media, digital media enable greater storage capacity, faster access and enable automated processing. They are ubiquitous in modern digital technologies. In summary, a computer-readable medium is any digital storage from which data can be retrieved and interpreted by computers.

The description of the subject application has been presented for purposes of illustration and description but is not intended to be exhaustive or limited to the application in the form disclosed. The embodiments were chosen and described to better explain the principles of the application and the practical application, and to enable the skilled person to understand the application for various embodiments with various modifications as are suited to the particular use contemplated.

## Claims

1. A system (100) implemented by a computer for providing personalized
virtual digital dentition of a patient, the system (100) comprising:
- at least one memory (110) configured for storing
-- computer program instructions,
-- a plurality of predetermined reference cranio-facial vectors, each
--- being associated with at least one type of predetermined human personality trait, and
--- comprising a set of reference cranio-facial features,
-- a teeth database (111) comprising 2D digital images of individual teeth, each being associated with one type of tooth and at least one predetermined human personality trait, and
-- a first predetermined sequence that is representative of a type of tooth order, in which 2D digital images of individual teeth are to be considered for positioning on a 2D image
- processor-based acquiring means (120) configured for acquiring at least one digital image of the front face of the patient smiling such that at least a portion of the dentition of the patient can be seen,
- processor-based computer vision means (130) comprising
-- processor-based teeth detecting means (131) configured for detecting at least one tooth in the digital image of the front face of the patient,
-- processor-based cranio-facial detecting means (132) configured for
--- detecting a set of cranio-facial features of the patient based on the digital image of the front face of the patient, and
--- generating a patient cranio-facial vector based on the detected set of cranio-facial features,
-- processor-based comparing means (133) configured for
-- comparing the patient cranio-facial vector with the plurality of predetermined reference cranio-facial vectors, and
-- calculating a plurality of similarity measures, that are greater than a predetermined similarity threshold, each being representative of a matching similarity between the patient cranio-facial vector and one predetermined reference cranio-facial vector,
-- processor-based ranking means (134) configured for ranking the similarity measures according to a given ranking order, and
-- processor-based positioning means (135) configured for overlaying, on the digital image of the front face of the patient, the detected tooth with the corresponding 2D digital image of individual tooth, based on the first predetermined sequence and the ranked similarity measures, thereby generating a first personalized virtual 2D digital dentition of the patient.

2. The system (100) of claim 1, wherein
- the processor-based computer vision means (130) further comprise processor-based dental arch trajectory detecting means (136) configured for detecting a trajectory of the dental arch of the patient in the digital image of the front face of the patient, and
- the processor-based positioning means (135) is further configured for modifying at least one parameter of the dental arch trajectory of the patient based on at least one similarity measure,
and wherein the processor-based positioning means (135) overlay the detected tooth with the corresponding 2D digital image of individual tooth, along the modified dental arch trajectory of the patient.

3. The system (100) of any one of claims 1 to 2, wherein the processor-based teeth detecting means (131) are further configured for detecting a mouth opening boundary region formed between the upper lip, the lower lip, the oral commissures of the mouth of the patient,
and wherein the processor-based teeth detecting means (131) detect the tooth within the mouth opening boundary region.

4. The system (100) of claim 3, further comprising a user interface (140) configured for allowing, through user interaction, digital modification of at least one point defining the mouth opening boundary region.

5. The system (100) of any one of claims 1 to 4, wherein the processor-based comparing means (133) comprise a machine learning classifier which is trained using, as training data, a plurality of sets of cranio-facial features that have been extracted from a plurality of images of the front face of human faces, the machine learning classifier being configured for
- predicting the likelihood that the set of cranio-facial features of a human face is similar to each of the set of reference cranio-facial features of the plurality of predetermined reference cranio-facial vectors, and
- outputting similarity measures representative of a matching similarity between an inputted set of cranio-facial features of a human face and each of the set of reference cranio-facial features of the plurality of predetermined reference cranio-facial vectors.

6. The system (100) of any one of claims 1 to 5, wherein
- the teeth database (111) further comprises 3D digital images of individual teeth, each being associated with one type of tooth and at least one predetermined human personality trait,
- the memory (110) is further configured for storing a second predetermined sequence that is representative of a type of tooth order, in which 3D digital images of individual teeth are to be considered for positioning on a 2D image, and
- the processor-based positioning means (135) are further configured for overlaying, on the digital image of the front face of the patient, the detected tooth with a 2D digital representation of a cross-section through the corresponding 3D digital image of individual tooth, based on the second predetermined sequence and the ranked similarity measures, thereby generating a second personalized virtual 2D digital dentition of the patient.

7. The system (100) of claim 6, wherein
- the processor-based acquiring means (120) are further configured for acquiring a 3D intraoral scan of the dentition of the patient,
- the processor-based teeth detecting means (131) are further configured for detecting at least one tooth in the 3D intraoral scan,
- within the teeth database (111), each 3D digital image is associated with one corresponding 2D digital image, and
- the processor-based positioning means (135) are further configured for
-- overlaying the first or second personalized virtual 2D digital dentition over the 3D intraoral scan, and
-- replacing the tooth detected in the 3D intraoral scan by the 3D digital image associated with the first or second personalized virtual 2D digital dentition, thereby generating a personalized virtual 3D digital dentition.

8. A method (200) of training a classifier for predicting the likelihood that a set of cranio-facial features of a human face is similar to each of a set of reference cranio-facial features of a plurality of predetermined reference cranio-facial vectors, the method (200) comprising, with a processor,
- collecting (210) a plurality of sets of cranio-facial training features, each being associated with at least an image of the front face of a human,
- generating (220) a plurality of cranio-facial training vectors based on the plurality of sets of cranio-facial training features,
- obtaining (230) a plurality of predetermined reference cranio-facial vectors, each
-- being associated with at least one type of predetermined human personality trait, and
-- comprising a set of reference cranio-facial features,
- calculating (240) a plurality of similarity measures, each being representative of a matching similarity between one set of cranio-facial training features and one predetermined reference cranio-facial vector, and
- applying (250) the plurality of sets of cranio-facial training features and the plurality of similarity measures to a machine-learning algorithm to generate a trained classifier configured for outputting similarity measures representative of a matching similarity between, on the one hand, an input cranio-facial vector, and on the other hand, each of the plurality of predetermined reference cranio-facial vectors.

9. The method (200) of claim 8, wherein the similarity measures correspond to matching percentages.

10. The method (200) of any one of claims 8 to 9, wherein the classifier is configured for outputting the similarity measures that are greater than a predetermined similarity threshold.

11. The method (200) of any one of claims 8 to 10, wherein the machine learning algorithm comprises a machine learning algorithm selected among random forests, support vector machine and neural networks.

12. The method (200) of any one of claims 8 to 11, wherein each set of cranio-facial training features comprises a combination of at least two measurable characteristics that can be derived from a substantially front face view of an individual.

13. The method (200) of any one of claims 8 to 12, wherein the collected plurality of sets of cranio-facial training features comprises a plurality of
- positive sets of cranio-facial training features which have been identified as being associated with at least one type of predetermined human personality trait, and
- negative sets of cranio-facial training features which have been identified as not being associated with at least one type of predetermined human personality trait.

14. The method (200) of claim 13, wherein the number of positive sets of cranio-facial training features is substantially equal to the number of negative sets of cranio-facial training features.

15. A computer-readable medium having stored thereon computer instructions which when executed, by a processor, perform a method (200) for training a classifier according to any one of claims 8 to 14.

## Patentansprüche

1. System (100), implementiert durch einen Computer, zum Bereitstellen eines personalisierten virtuellen digitalen Gebisses eines Patienten, wobei das System (100) umfasst:
- mindestens einen Speicher (110), der konfiguriert ist zum Speichern von
-- Computerprogrammanweisungen,
-- einer Vielzahl von vorbestimmten kraniofazialen Referenzvektoren, die jeweils
--- mindestens einer Art von vorbestimmtem menschlichen Persönlichkeitszug zugeordnet sind, und
--- einen Satz von kraniofazialen Referenzmerkmalen umfassen,
-- einer Zahndatenbank (111) umfassend digitale 2D-Bilder einzelner Zähne, die jeweils einem Zahntyp und mindestens einem vorbestimmten menschlichen Persönlichkeitszug zugeordnet sind, und
-- einer ersten vorbestimmten Sequenz, die für eine Art von Zahnordnung repräsentativ ist, in der digitale 2D-Bilder einzelner Zähne für die Positionierung auf einem 2D-Bild zu berücksichtigen sind
- prozessorbasierte Erfassungsmittel (120), die so konfiguriert sind, dass sie mindestens ein digitales Bild des Frontgesichts des lächelnden Patienten so erfassen, dass mindestens ein Teil des Gebisses des Patienten zu sehen ist,
- prozessorbasierte Computer-Vision-Mittel (130) umfassend
-- prozessorbasierte Zahnerkennungsmittel (131), die so konfiguriert ist, dass sie mindestens einen Zahn in dem digitalen Bild des Frontgesichts des Patienten erkennen,
-- prozessorbasierte kraniofaziale Erkennungsmittel (132), die konfiguriert sind zum
--- Erkennen eines Satzes von kraniofazialen Merkmalen des Patienten auf der Grundlage des digitalen Bildes des Frontgesichts des Patienten, und
--- Erzeugen eines kraniofazialen Vektors für den Patienten auf der Grundlage des erkannten Satzes von kraniofazialen Merkmalen,
-- prozessorbasierte Vergleichsmittel (133), die konfiguriert sind zum
-- Vergleichen des kraniofazialen Vektors für den Patienten mit einer Vielzahl von vorbestimmten kraniofazialen Referenzvektoren, und
-- Berechnen einer Vielzahl von Ähnlichkeitsmaßen, die größer als ein vorbestimmter Ähnlichkeitsschwellenwert sind, wobei jedes Maß für eine übereinstimmende Ähnlichkeit zwischen dem kraniofazialen Vektor für den Patienten und einem vorbestimmten kraniofazialen Referenzvektor repräsentativ ist,
-- prozessorbasierte Rangordnungsmittel (134), die so konfiguriert sind, dass sie die Ähnlichkeitsmaße gemäß einer gegebenen Rangordnung ordnen, und
-- prozessorbasierte Positionierungsmittel (135), die so konfiguriert ist, dass sie auf dem digitalen Bild des Frontgesichts des Patienten den erkannten Zahn mit dem entsprechenden digitalen 2D-Bild eines einzelnen Zahns überlagern, und zwar auf der Grundlage der ersten vorbestimmten Sequenz und der geordneten Ähnlichkeitsmaße, wodurch ein erstes personalisiertes virtuelles digitales 2D-Gebiss des Patienten erzeugt wird.

2. System (100) nach Anspruch 1, wobei
- die prozessorbasierten Computer-Vision-Mittel (130) ferner prozessorbasierte Zahnbogentrajektorie-Erkennungsmittel (136) umfassen, die so konfiguriert sind, dass sie eine Trajektorie des Zahnbogens des Patienten in dem digitalen Bild des Frontgesichts des Patienten erkennen, und
- die prozessorgestützten Positionierungsmittel (135) ferner so konfiguriert ist, dass sie mindestens einen Parameter der Zahnbogentrajektorie des Patienten auf der Grundlage von mindestens einem Ähnlichkeitsmaß modifizieren,
und wobei die prozessorbasierten Positionierungsmittel (135) den erkannten Zahn mit dem entsprechenden digitalen 2D-Bild des einzelnen Zahns entlang der modifizierten Zahnbogentrajektorie des Patienten überlagern.

3. System (100) nach einem der Ansprüche 1 bis 2, wobei die prozessorbasierten Zahnerkennungsmittel (131) ferner so konfiguriert sind, dass sie einen Grenzbereich der Mundöffnung erkennen, der zwischen der Oberlippe, der Unterlippe und den Mundwinkeln des Mundes des Patienten gebildet wird,
und wobei die prozessorbasierten Zahnerkennungsmittel (131) den Zahn in dem Grenzbereich der Mundöffnung erkennen.

4. System (100) nach Anspruch 3, ferner umfassend eine Benutzerschnittstelle (140), die so konfiguriert ist, dass sie durch Benutzerinteraktion eine digitale Modifikation von mindestens einem Punkt ermöglicht, der den Grenzbereich der Mundöffnung definiert.

5. System (100) nach einem der Ansprüche 1 bis 4, wobei die prozessorbasierten Vergleichsmittel (133) einen maschinell lernenden Klassifikator umfassen, der trainiert wird, indem als Trainingsdaten eine Vielzahl von Sätzen von kraniofazialen Merkmalen verwendet wird, die aus einer Vielzahl von Bildern des Frontgesichts menschlicher Gesichter extrahiert wurden, wobei der maschinell lernende Klassifikator konfiguriert ist zum
- Vorhersagen der Wahrscheinlichkeit, dass der Satz von kraniofazialen Merkmalen eines menschlichen Gesichts jedem von dem Satz von kraniofazialen Referenzmerkmalen aus der Vielzahl von vorbestimmten kraniofazialen Referenzvektoren ähnlich ist, und
- Ausgeben von Ähnlichkeitsmaßen, die eine übereinstimmende Ähnlichkeit zwischen einem eingegebenen Satz von kraniofazialen Merkmalen eines menschlichen Gesichts und jedem von dem Satz von kraniofazialen Referenzmerkmalen aus der Vielzahl von vorbestimmten kraniofazialen Referenzvektoren darstellen.

6. System (100) nach einem der Ansprüche 1 bis 5, wobei
- die Zahndatenbank (111) ferner digitale 3D-Bilder einzelner Zähne umfasst, die jeweils einem Zahntyp und mindestens einem vorbestimmten menschlichen Persönlichkeitszug zugeordnet sind,
- der Speicher (110) ferner so konfiguriert ist, dass er eine zweite vorbestimmte Sequenz speichert, die für eine Art von Zahnordnung repräsentativ ist, in der digitale 3D-Bilder einzelner Zähne für die Positionierung auf einem 2D-Bild zu berücksichtigen sind, und
-- die prozessorbasierten Positionierungsmittel (135) ferner so konfiguriert sind, dass sie auf dem digitalen Bild des Frontgesichts des Patienten den erkannten Zahn mit einer digitalen 2D-Darstellung eines Querschnitts durch das entsprechende digitale 3D-Bild eines einzelnen Zahns überlagern, und zwar auf der Grundlage der zweiten vorbestimmten Sequenz und der geordneten Ähnlichkeitsmaße, wodurch ein zweites personalisiertes virtuelles digitales 2D-Gebiss des Patienten erzeugt wird.

7. System (100) nach Anspruch 6, wobei
- die prozessorbasierten Erfassungsmittel (120) ferner so konfiguriert sind, dass sie einen intraoralen 3D-Scan des Gebisses des Patienten erfassen,
- die prozessorbasierten Zahnerkennungsmittel (131) ferner so konfiguriert sind, dass sie mindestens einen Zahn in dem intraoralen 3D-Scan erkennen,
- in der Zahndatenbank (111) jedes digitale 3D-Bild einem entsprechenden digitalen 2D-Bild zugeordnet ist, und
- die prozessorgestützten Positionierungsmittel (135) ferner konfiguriert zum
-- Überlagern des ersten oder des zweiten personalisierten virtuellen digitalen 2D-Gebisses über den intraoralen 3D-Scan, und
-- Ersetzen des in dem intraoralen 3D-Scan erkannten Zahns durch das digitale 3D-Bild, das dem ersten oder dem zweiten personalisierten virtuellen digitalen 2D-Gebiss zugeordnet ist, wodurch ein personalisiertes virtuelles digitales 3D-Gebiss erzeugt wird.

8. Verfahren (200) des Trainieren eines Klassifikators zum Vorhersagen der Wahrscheinlichkeit, dass ein Satz von kraniofazialen Merkmalen eines menschlichen Gesichts jedem von dem Satz von kraniofazialen Referenzmerkmalen aus einer Vielzahl von vorbestimmten kraniofazialen Referenzvektoren ähnlich ist, das Verfahren (200) umfassend, mit einem Prozessor,
- das Sammeln (210) einer Vielzahl von Sätzen von kraniofazialen Trainingsmerkmalen, von denen jedes mindestens einem Bild des Frontgesichts eines Menschen zugeordnet ist,
- das Erzeugen (220) einer Vielzahl von kraniofazialen Trainingsvektoren auf der Grundlage der Vielzahl von Sätzen von kraniofazialen Trainingsmerkmalen,
- das Erlangen (230) einer Vielzahl von vorbestimmten kraniofazialen Referenzvektoren, die jeweils
-- mindestens einer Art von vorbestimmtem menschlichen Persönlichkeitszug zugeordnet sind, und
-- einen Satz von kraniofazialen Referenzmerkmalen umfassen,
- das Berechnen (240) einer Vielzahl von Ähnlichkeitsmaßen, von denen jedes für eine übereinstimmende Ähnlichkeit zwischen einem Satz von kraniofazialen Trainingsmerkmalen und einem vorbestimmten kraniofazialen Referenzvektor repräsentativ ist, und
- das Anwenden (250) der Vielzahl von Sätzen von kraniofazialen Trainingsmerkmalen und der Vielzahl von Ähnlichkeitsmaßen auf einen Algorithmus zum maschinellen Lernen, um einen trainierten Klassifikator zu erzeugen, der zum Ausgeben von Ähnlichkeitsmaßen konfiguriert ist, die eine übereinstimmende Ähnlichkeit zwischen einerseits einem eingegebenen kraniofazialen Vektor und andererseits jedem der Vielzahl von vorbestimmten kraniofazialen Referenzvektoren darstellen.

9. Verfahren (200) nach Anspruch 8, wobei die Ähnlichkeitsmaße den Übereinstimmungsprozentsätzen entsprechen.

10. Verfahren (200) nach einem der Ansprüche 8 bis 9, wobei der Klassifikator so konfiguriert ist, dass er die Ähnlichkeitsmaße ausgibt, die größer als ein vorgegebener Ähnlichkeitsschwellenwert sind.

11. Verfahren (200) nach einem der Ansprüche 8 bis 10, wobei der Algorithmus zum maschinellen Lernen einen Algorithmus zum maschinellen Lernen umfasst, der ausgewählt ist aus Random Forest, Support Vector Machine und neuronalen Netzen.

12. Verfahren (200) nach einem der Ansprüche 8 bis 11, wobei jeder Satz von kraniofazialen Trainingsmerkmalen eine Kombination von mindestens zwei messbaren Merkmalen umfasst, die von einer im Wesentlichen frontalen Gesichtsansicht eines Individuums abgeleitet werden können.

13. Verfahren (200) nach einem der Ansprüche 8 bis 12, wobei die gesammelte Vielzahl von Sätzen von kraniofazialen Trainingsmerkmalen eine Vielzahl umfasst von
- positiven Sätzen von kraniofazialen Trainingsmerkmalen, die identifiziert wurden als mindestens einer Art von vorbestimmtem menschlichem Persönlichkeitszug zugeordnet, und
- negativen Sätzen von kraniofazialen Trainingsmerkmalen, die identifiziert wurden als nicht mindestens einer Art von vorbestimmtem menschlichem Persönlichkeitszug zugeordnet.

14. Verfahren (200) nach Anspruch 13, wobei die Anzahl von positiven Sätzen von kraniofazialen Trainingsmerkmalen im Wesentlichen gleich der Anzahl von negativen Sätzen von kraniofazialen Trainingsmerkmalen ist.

15. Computerlesbares Medium, auf dem Computeranweisungen gespeichert sind, die, wenn sie von einem Prozessor ausgeführt werden, ein Verfahren (200) zum Trainieren eines Klassifikators gemäß einem der Ansprüche 8 bis 14 durchführen.

## Revendications

1. Système (100) mis en œuvre par un ordinateur pour fournir une dentition numérique virtuelle personnalisée d'un patient, le système (100) comprenant :
- au moins un mémoire (110) configurée pour stocker
-- des instructions de programme d'ordinateur,
-- une pluralité de vecteurs crânio-faciaux de référence prédéterminés, chacun
--- étant associé à au moins un type de trait de personnalité humaine prédéterminé, et
--- comprenant un ensemble de caractéristiques crânio-faciales de référence,
-- une base de données de dents (111) comprenant des images numériques 2D de dents individuelles, chacune étant associée à un type de dent et à au moins un trait de personnalité humaine prédéterminé, et
-- une première séquence prédéterminée qui est représentative d'un type d'ordre de dent, dans laquelle des images numériques 2D de dents individuelles doivent être prises en compte pour un positionnement sur une image 2D
- des moyens d'acquisition basés sur processeur (120) configurés pour acquérir au moins une image numérique de l'avant du visage du patient souriant de sorte qu'au moins une partie de la dentition du patient puisse être observée,
- des moyens de visionique basés sur processeur (130) comprenant
-- des moyens de détection de dents basés sur processeur (131) configurés pour détecter au moins une dent dans l'image numérique de l'avant du visage du patient,
-- des moyens de détection crânio-faciaux basés sur processeur (132) configurés pour
--- détecter un ensemble de caractéristiques crânio-faciales du patient sur la base de l'image numérique de l'avant du visage du patient, et
--- générer un vecteur crânio-facial de patient sur la base de l'ensemble de caractéristiques crânio-faciales détecté,
-- des moyens de comparaison basés sur processeur (133) configurés pour
-- comparer le vecteur crânio-facial de patient avec la pluralité de vecteurs crânio-faciaux de référence prédéterminés, et
-- calculer une pluralité de mesures de similarité, qui sont supérieures à un seuil de similarité prédéterminé, chacune étant représentative d'une similarité de concordance entre le vecteur crânio-facial de patient et un vecteur crânio-facial de référence prédéterminé,
-- des moyens de classement basés sur processeur (134) configurés pour classer les mesures de similarité selon un ordre de classement donné, et
-- des moyens de positionnement basés sur processeur (135) configurés pour superposer, sur l'image numérique de l'avant du visage du patient, la dent détectée avec l'image numérique 2D correspondante de dent individuelle, sur la base de la première séquence prédéterminée et des mesures de similarité classées, ce qui génère une première dentition numérique 2D virtuelle personnalisée du patient.

2. Système (100) selon la revendication 1, dans lequel
- les moyens de visionique basés sur processeur (130) comprennent en outre des moyens de détection de trajectoire d'arcade dentaire basés sur processeur (136) configurés pour détecter une trajectoire de l'arcade dentaire du patient dans l'image numérique de l'avant du visage du patient, et
- les moyens de positionnement basés sur processeur (135) sont en outre configurés pour modifier au moins un paramètre de la trajectoire d'arcade dentaire du patient sur la base d'au moins une mesure de similarité,
et dans lequel les moyens de positionnement basés sur processeur (135) superposent la dent détectée avec l'image numérique 2D correspondante de dent individuelle, le long de la trajectoire d'arcade dentaire modifiée du patient.

3. Système (100) selon l'une quelconque des revendications 1 à 2, dans lequel les moyens de détection de dents basés sur processeur (131) sont en outre configurés pour détecter une région de limite d'ouverture de bouche formée entre la lèvre supérieure, la lèvre inférieure et les commissures orales de la bouche du patient,
et dans lequel les moyens de détection de dents basés sur processeur (131) détectent la dent au sein de la région de limite d'ouverture de bouche.

4. Système (100) selon la revendication 3, comprenant en outre une interface utilisateur (140) configurée pour permettre, par le biais d'une interaction d'utilisateur, une modification numérique d'au moins un point définissant la région de limite d'ouverture de bouche.

5. Système (100) selon l'une quelconque des revendications 1 à 4, dans lequel les moyens de comparaison basés sur processeur (133) comprennent un classificateur à apprentissage automatique qui est entraîné à l'aide, en tant que données d'entraînement, d'une pluralité d'ensembles de caractéristiques crânio-faciales qui ont été extraites d'une pluralité d'images de l'avant du visage de visages humains, le classificateur à apprentissage automatique étant configuré pour
- prédire la probabilité que l'ensemble de caractéristiques crânio-faciales d'un visage humain soit similaire à chacune de l'ensemble de caractéristiques crânio-faciales de référence de la pluralité de vecteurs crânio-faciaux de référence prédéterminés, et
- délivrer en sortie des mesures de similarité représentatives d'une similarité de concordance entre un ensemble entré de caractéristiques crânio-faciales d'un visage humain et chacune de l'ensemble de caractéristiques crânio-faciales de référence de la pluralité de vecteurs crânio-faciaux de référence prédéterminés.

6. Système (100) selon l'une quelconque des revendications 1 à 5, dans lequel
- la base de données de dents (111) comprend en outre des images numériques 3D de dents individuelles, chacune étant associée à un type de dent et à au moins un trait de personnalité humaine prédéterminé,
- la mémoire (110) est en outre configurée pour stocker une deuxième séquence prédéterminée qui est représentative d'un type d'ordre de dent, dans laquelle des images numériques 3D de dents individuelles doivent être prises en compte pour un positionnement sur une image 2D, et
- les moyens de positionnement basés sur processeur (135) sont en outre configurés pour superposer, sur l'image numérique de l'avant du visage du patient, la dent détectée avec une représentation numérique 2D d'une section transversale à travers l'image numérique 3D correspondante de dent individuelle, sur la base de la deuxième séquence prédéterminée et des mesures de similarité classées, ce qui génère une deuxième dentition numérique 2D virtuelle personnalisée du patient.

7. Système (100) selon la revendication 6, dans lequel
- les moyens d'acquisition basés sur processeur (120) sont en outre configurés pour acquérir une numérisation intra-orale 3D de la dentition du patient,
- les moyens de détection de dents basés sur processeur (131) sont en outre configurés pour détecter au moins une dent dans la numérisation intra-orale 3D,
- au sein de la base de données de dents (111), chaque image numérique 3D est associée à une image numérique 2D correspondante, et
- les moyens de positionnement basés sur processeur (135) sont en outre configurés pour
-- superposer la première ou la deuxième dentition numérique 2D virtuelle personnalisée sur la numérisation intra-orale 3D, et
-- remplacer la dent détectée dans la numérisation intra-orale 3D par l'image numérique 3D associée à la première ou à la deuxième dentition numérique 2D virtuelle personnalisée, ce qui génère une dentition numérique 3D virtuelle personnalisée.

8. Procédé (200) d'entraînement d'un classificateur pour prédire la probabilité qu'un ensemble de caractéristiques crânio-faciales d'un visage humain soient similaires à chacune d'un ensemble de caractéristiques crânio-faciales de référence d'une pluralité de vecteurs crânio-faciaux de référence prédéterminés, le procédé (200) comprenant, avec un processeur,
- la collecte (210) d'une pluralité d'ensembles de caractéristiques d'entraînement crânio-faciales, chacune étant associée à au moins une image de l'avant du visage d'un humain,
- la génération (220) d'une pluralité de vecteurs d'entraînement crânio-faciaux sur la base de la pluralité d'ensembles de caractéristiques d'entraînement crânio-faciales,
- l'obtention (230) d'une pluralité de vecteurs crânio-faciaux de référence prédéterminés, chacun
-- étant associé à au moins un type de trait de personnalité humaine prédéterminé, et
-- comprenant un ensemble de caractéristiques crânio-faciales de référence,
- le calcul (240) d'une pluralité de mesures de similarité, chacune étant représentative d'une similarité de concordance entre un ensemble de caractéristiques d'entraînement crânio-faciales et un vecteur crânio-facial de référence prédéterminé, et
- l'application (250) de la pluralité d'ensembles de caractéristiques d'entraînement crânio-faciales et de la pluralité de mesures de similarité à un algorithme d'apprentissage automatique pour générer un classificateur entraîné configuré pour délivrer en sortie des mesures de similarité représentatives d'une similarité de concordance entre, d'une part, un vecteur crânio-facial d'entrée, et d'autre part, chacun de la pluralité de vecteurs crânio-faciaux de référence prédéterminés.

9. Procédé (200) selon la revendication 8, dans lequel les mesures de similarité correspondent à des pourcentages de concordance.

10. Procédé (200) selon l'une quelconque des revendications 8 à 9, dans lequel le classificateur est configuré pour délivrer en sortie les mesures de similarité qui sont supérieures à un seuil de similarité prédéterminé.

11. Procédé (200) selon l'une quelconque des revendications 8 à 10, dans lequel l'algorithme d'apprentissage automatique comprend un algorithme d'apprentissage automatique sélectionné parmi les forêts aléatoires, les machines à vecteurs de support et les réseaux de neurones.

12. Procédé (200) selon l'une quelconque des revendications 8 à 11, dans lequel chaque ensemble de caractéristiques d'entraînement crânio-faciales comprend une combinaison d'au moins deux propriétés mesurables qui peuvent être déduites d'une vue sensiblement de l'avant du visage d'un individu.

13. Procédé (200) selon l'une quelconque des revendications 8 à 12, dans lequel la pluralité collectée d'ensembles de caractéristiques d'entraînement crânio-faciales comprend une pluralité
- d'ensembles positifs de caractéristiques d'entraînement crânio-faciales qui ont été identifiés comme étant associés à au moins un type de trait de personnalité humaine prédéterminé, et
- d'ensembles négatifs de caractéristiques d'entraînement crânio-faciales qui ont été identifiés comme n'étant pas associés à au moins un type de trait de personnalité humaine prédéterminé.

14. Procédé (200) selon la revendication 13, dans lequel le nombre d'ensembles positifs de caractéristiques d'entraînement crânio-faciales est sensiblement égal au nombre d'ensembles négatifs de caractéristiques d'entraînement crânio-faciales.

15. Support lisible par ordinateur ayant, stockées sur celui-ci, des instructions d'ordinateur qui, lors de leur exécution par un processeur, réalisent un procédé (200) pour l'entraînement d'un classificateur selon l'une quelconque des revendications 8 à 14.
